# EUROPÄISCHE PATENTSCHRIFT

(11) **EP 0 555 645 B1**
(45) Veröffentlichungstag und Bekanntmachung des Hinweises auf die Patenterteilung: **15.04.1998**
(21) Anmeldenummer: 93100405.5
(22) Anmeldetag: 13.01.1993
(51) Int. Cl.: A61C 19/04, A61B 5/00, G01N 21/64

(54) **Einrichtung zum Erkennen von Karies an Zähnen**
Device for detecting dental caries
Dispositif pour détecter la carie dentaire

(30) Priorität: 14.01.1992 DE 4200741
(43) Veröffentlichungstag der Anmeldung: 18.08.1993
(73) Patentinhaber: KALTENBACH & VOIGT GMBH & CO., 88400 Biberach (DE)
(72) Erfinder: Hibst, Raimund, Dr.rer.nat., W-7904 Erbach (DE); König, Karsten, Dr.rer.nat., W-7906 Blaustein (DE)
(74) Vertreter: Schmidt-Evers, Jürgen, Dipl.-Ing.

(56) Entgegenhaltungen:
- EP-A- 0 073 180
- EP-A- 0 113 152
- DE-A- 3 347 516
- GB-A- 2 058 343
- US-A- 4 479 499

## Beschreibung

Die Erfindung betrifft eine Einrichtung zum Erkennen von Karies an Zähnen nach dem Oberbegriff des Anspruchs 1.

In der Zeitschrift SPIE, Vol. 907, Laser Surgery: Characterization and Therapeutics, 1988, S. Albin et al., "Laser Induced Fluorescence of Dental Caries", Seiten 96 bis 98, ist eine Einrichtung zum Erkennen von Karies an Zähnen beschrieben. Bei dieser bekannten Einrichtung sendet ein Laser eine monochromatische Strahlung auf den Zahn aus, die eine Fluorzeszenzstrahlung anregt. Von kariösen Stellen des Zahns geht eine für Karies charakteristische Fluoreszenzstrahlung aus, die sich in ihrer Intensität und spektralen Verteilung von der zurückgestrahlten Strahlung eines gesunden Zahns unterscheidet. Die zurückgestrahlte Strahlung wird durch ein Filter beobachtet. Die von Karies befallenen Stellen des Zahns erscheinen bei der Beobachtung als dunkle Flecken.

Diese bekannte Einrichtung ist für den Laborbetrieb vorgesehen, bei dem optimale Versuchsbedingungen vorhanden und brauchbare Versuchsergebnisse auch noch bei geringer Empfindlichkeit der Einrichtung erzielbar sind. Für einen praxisnahen Einsatz bei der Zahndiagnose ist es jedoch erforderlich, die Empfindlichkeit der Einrichtung zum Erkennen von Karies erheblich zu steigern. Außerdem ist die bekannte Einrichtung nur schwer handhabbar, so daß diese als Diagnosemittel bei der Anwendung am Menschen oder an Tieren nicht geeignet ist.

Eine Einrichtung der eingangs angegebenen Art, die dem Oberbegriff des Anspruchs 1 zugrunde liegt, ist in der US-A-4 479 499 beschrieben. Bei dieser bekannten Einrichtung ist ein gemeinsamer Lichtleiter für die von der Beleuchtungseinrichtung ausgehende Strahlung und für die vom Zahn zurückgestrahlte Strahlung vorgesehen. Vom hinteren Endbereich des Lichtleiters zweigen zwei Lichtleiterzweige ab, durch die die zurückgestrahlte Strahlung jeweils über einen Filter und einen Fotodetektor einer Differenzauswerteschaltung zugeführt wird, deren Signale auf einem Bildschirm dargestellt werden könne. Bei einer solchen Lichtleiterabzweigung ist nicht nur die Bauweise, sondern auch die im Bereich der Abzweigung stattfindende Lichtabzweigung problematisch.

Der Erfindung liegt deshalb die Aufgabe zugrunde, die Abzweigung der zurückgestrahlten Strahlung zu verbessern.

Diese Aufgabe wird durch die Merkmale des Anspruchs 1 gelöst.

Bei der erfindungsgemäßen Einrichtung ist der Lichtleiter auf der Seite der Beleuchtungseinrichtung mit einem Teilerspiegel versehen, dessen Teilerfläche gegen die optische Achse des Lichtleiters geneigt ist, und der die zurückgestrahlte Strahlung seitlich herausreflektiert.

Auch bei der Erfindung wird dem zu untersuchenden Zahn die Strahlung über einen Lichtleiter zugeführt. Ein solcher Lichtleiter kann starr oder flexibel, an seinem dem Zahn zugewandten Ende mit optischen Mitteln zur gezielten Strahlführung ausgestattet und/oder in seinen Abmessungen an den Mundbereich des Patienten sowie an den Zahn angepaßt sein. An dem Lichtleiter können in bekannter Weise austauschbare Vorsätze, beispielsweise Umlenkspiegel oder Linsen, angebracht sein, die eine Untersuchung des Zahns erleichtern. Die Verwendung des Lichtleiters ermöglicht es also, die Strahlung gezielt dem zu untersuchenden Bereich des Zahns oder der Zähne zuzuführen. Dadurch kann die Einrichtung nach der Erfindung flexibel an verschiedene Erfordernisse in der täglichen Praxis bei der Karieserkennung an Zähnen von Tieren und Menschen angepaßt werden.

Eine Weiterbildung der Erfindung bezieht sich darauf, den Wellenlängenbereich der vom Filter durchgelassenen Strahlung nach unten zu beschränken. Dies bedeutet, daß vorwiegend die Fluoreszenzstrahlung ausgefiltert wird und die störende Umgebungsstrahlung mit kurzen Wellenlängen ausgeblendet wird. Dadurch bilden sich die kariösen Stellen des Zahns als helle Flecken ab, die sich deutlich vom Hintergrund abheben. Ein kariöser Krankheitsbefund kann so mit hoher Genauigkeit und Zuverlässigkeit erkannt werden. Die Einrichtung nach der Erfindung eignet sich daher gut für die Frühdiagnose von Karies.

Bei praktischen Versuchen hat es sich gezeigt, daß die Karieserreger bei Einstrahlung von Licht im angegebenen Wellenlängenbereich empfindlich reagieren, und bei hinreichend starker Intensität der Strahlung absterben. Mit der Einrichtung nach der Erfindung ist es also möglich, eine Kariestherapie an Zähnen unter gleichzeitiger Beobachtung des Orts der Behandlung durchzuführen. Auf diese Weise wird eine nicht invasive, schmerzfreie, selektiv wirkende, extrem schonende Kariestherapie möglich, die sich besonders zur Behandlung von Karies im Frühstadium und zur Prophylaxe eignet.

Ein bevorzugtes Ausführungsbeispiel ist dadurch gekennzeichnet, daß das Filter die zurückgestrahlte Strahlung im Wellenlängenbereich von 620 bis 720 nm durchläßt. In diesem Wellenlängenbereich hat das von Zahnkaries emittierte Fluoreszenzspektrum Maxima, d.h. die Ausbeute an Fluoreszenzstrahlung aus umgesetzter Anregungsenergie ist hoch. Dadurch wird die Ansprechempfindlichkeit der Einrichtung auf Karies weiter erhöht. Außerdem ist bei diesem Wellenlängenbereich der Abstand zum Wellenlängenbereich der Anregungsstrahlung im Wellenlängenbereich von 360 bis 580 nm groß, so daß diese weitgehend unterdrückt wird und das Ergebnis nicht verfälschen kann. Die kariösen Stellen können dadurch mit hohem Kontrast erkannt werden.

Ein weiteres Ausführungsbeispiel ist dadurch gekennzeichnet, daß die Beleuchtungseinrichtung Strahlung im Wellenlängenbereich 360 bis 420 nm aussendet.

In diesem Wellenlängenbereich ist die Intensität der zurückgestrahlten Strahlung besonders hoch, so daß eine sehr empfindliche Detektion kariöser Stellen möglich ist. Bei einer Wellenlänge der eingestrahlten Strahlung im Bereich um 406 nm ist die Empfindlichkeit maximal. Ferner ist bei diesem Wellenlängenbereich der Abstand zum Wellenlängenbereich oberhalb von 620 nm der vom Zahn emittierten Strahlung groß, so daß die eingestrahlte Strahlung weitgehend unterdrückt wird und das Ergebnis nicht verfälscht. Die kariösen Stellen des Zahns können dadurch gut erkannt werden.

Ein anderes Ausführungsbeispiel kennzeichnet sich dadurch aus, daß die Beleuchtungseinrichtung Strahlung im Wellenlängenbereich 470 bis 580 nm aussendet.

Die eingestrahlte Strahlung hat bei diesem Wellenlängenbereich eine erhöhte Eindringtiefe und kann in tiefe Regionen des Zahnes eindringen oder Zahnbeläge durchdringen. Dadurch wird es möglich, verdeckte Krankheitsherde aufzuspüren. Vorzugsweise wird zur Einstrahlung eine Strahlungsquelle vorgesehen, die annähernd monochromatische Strahlung der Wellenlänge um 470, 500, 540 oder 580 nm aussendet.

Zur quantitativen Auswertung der zurückgestrahlten Strahlung ist in einem weiteren Ausführungsbeispiel dem Filter in Richtung der zurückgestrahlten Strahlung gesehen ein Detektor nachgeschaltet, der die ihm zugeführte Strahlung in ein erstes elektrisches Signal umwandelt. Dieses Signal, das in bekannter Weise angezeigt werden kann, ist annähernd proportional der vom Detektor empfangenen Strahlungsintensität. Es kann daher zur quantitativen Beurteilung des Ausmaßes des festgestellten Kariesbefundes herangezogen werden.

Praktische Untersuchungen haben gezeigt, daß sich die jeweiligen Spektren der zurückgestrahlten Strahlung gesunder und kariös veränderter Hartsubstanz des Zahns signifikant unterscheiden. Bei einem Ausführungsbeispiel wird dieser Effekt ausgenutzt. Bei dieser Weiterbildung ist ein weiteres Filter vorgesehen, das die vom Zahn zurückgestrahlte Strahlung in einem weiteren, vom Durchlaß-Wellenlängenbereich des ersten Filters verschiedenen Wellenlängenbereich durchläßt, wobei der Detektor oder ein weiterer Detektor die ausgefilterte Strahlung in ein zweites elektrisches Signal umwandelt, das einem Quotientenbildner zugeführt wird, der aus dem ersten Signal und dem zweiten Signal einen Quotienten bildet, welcher als Maß für einen Kariesbefall dient.

Bei der Erprobung der Erfindung hat sich herausgestellt, daß das unterschiedliche Verhalten der zurückgestrahlten Strahlung eines kariösen und eines gesunden Zahns besonders deutlich hervortritt, wenn die Intensität der zurückgestrahlten Strahlung im Wellenlängenbereich von 620 bis 720 nm mit derjenigen im Bereich von 540 bis 560 nm verglichen wird. Der erstgenannte Wellenlängenbereich enthält Intensitätsmaxima der von Karies hervorgerufenen Fluoreszenzstrahlung, während im Wellenlängenbereich von 540 bis 560 nm kein auffälliges Verhalten vorhanden ist. Die Strahlungsintensität in diesem Bereich kann daher als Referenz herangezogen werden. Durch die Maßnahmen dieser Weiterbildung ist es möglich, ein objektives Urteil über den Krankheitsbefund abzugeben.

Ein weiterer Vorteil der Quotientenbildung liegt darin, daß das Ergebnis unabhängig von der Intensität der eingestrahlten Strahlung ist. Wenn sich die Intensität der eingestrahlten Strahlung beispielsweise infolge Alterung der Strahlquelle oder Stromversorgungsschwankungen ändert, so bleibt dennoch bei sonst gleichen Versuchsbedingungen der Quotient konstant. Dadurch wird die Anwendbarkeit der Erfindung in der Praxis weiter verbessert.

Ausführungsbeispiele der Erfindung werden im folgenden an Hand der Zeichnungen erläutert. Darin zeigen:
- Fig. 1: eine Einrichtung zur visuellen Diagnose von Zahnkaries,
- Fig. 2: eine andere Einrichtung, bei der die einfallende und die zurückgestrahlte Fluoreszenzstrahlung in Lichtleitern geführt werden,
- Fig. 3: eine Einrichtung mit einem Bildleiter,
- Fig. 4: Verläufe der Strahlungsintensität der zurückgestrahlten Strahlung über die Wellenlänge bei einem kariösen und einem gesunden Zahn,
- Fig. 5: eine Detektoreinrichtung, bei der in den Strahlengang ein Filterrad geschaltet ist,
- Fig. 6: eine Detektoreinrichtung mit aufgeteiltem Lichtleiter, und
- Fig. 7: eine Detektoreinrichtung zur Spektralanalyse der zurückgestrahlten Strahlung mit einem Reflexionsgitter.

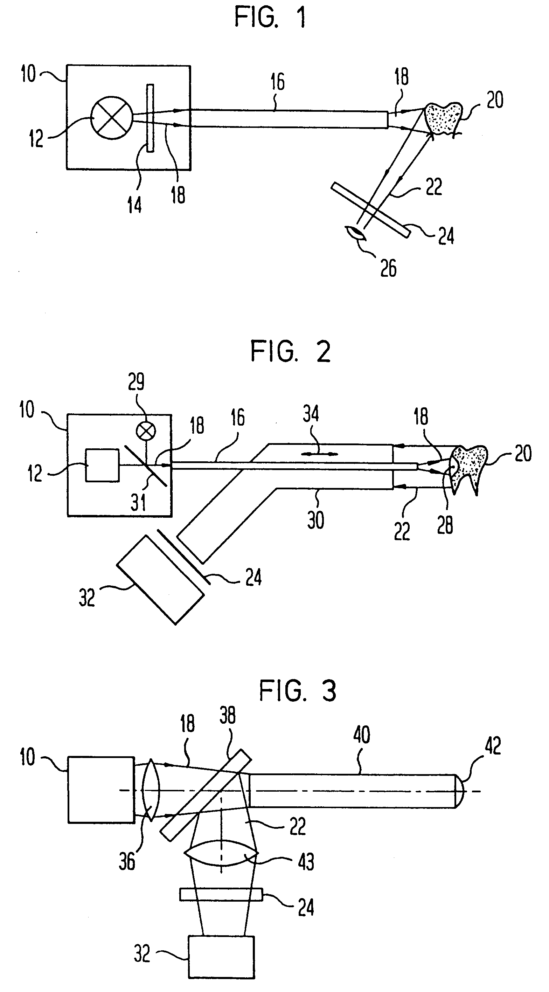

Die Fig. 1 und 2 zeigen nicht den vollständigen Gegenstand der Erfindung. Die kennzeichnenden Merkmale der Erfindung sind nur in Fig. 3 dargestellt.

In Fig. 1 ist der grundsätzliche Aufbau einer Einrichtung zum Erkennen und gegebenenfalls zum Behandeln von Karies an Zähnen dargestellt. Eine Beleuchtungseinrichtung 10 hat eine Strahlquelle 12, die Strahlung in einem Wellenlängenbereich von 360 bis 420 nm aussendet. Als Strahlquelle 12 kann eine Quecksilberdampflampe, ein Kryptonlaser, eine Halogenlampe oder ein Farbstofflaser verwendet werden. Zum Ausfiltern des gewünschten Wellenlängenbereichs ist vor die Strahlquelle 12 ein Strahlungsfilter 14 geschaltet.

Die Beleuchtungseinrichtung 10 ist mit einem Lichtleiter 16 versehen, der eine von der Strahlquelle 12 ausgesendete Strahlung 18 auf einen zu untersuchenden Zahn 20 lenkt. Die von der Oberfläche des Zahns 20 infolge von Reflexion und Fluoreszenz zurückgestrahlte Strahlung 22 wird durch ein Filter 24 gefiltert, das Strahlung mit Wellenlänge kleiner als 620 nm absorbiert oder reflektiert und Strahlung größerer Wellenlänge durchläßt. Das Filter 24 ist auf einem Brillengestell (nicht dargestellt) montiert, das von einer Person zur visuellen Beobachtung (angedeutet durch ein Auge 26) getragen wird. Die eingestrahlte Strahlung 18 regt in Bereichen des Zahns 20, die mit Karies befallen sind, eine charakteristische Fluoreszenzstrahlung an, deren spektralen Anteile mit Wellenlängen oberhalb der Durchlaßwellenlänge des Filters 24 auf das Auge 26 gelangen. Kariöse Stellen des Zahns 20 können somit beobachtet werden.

Die Einrichtung nach Fig. 1 kann grundsätzlich auch zur Kariestherapie eingesetzt werden. Hierzu ist eine Strahlquelle 12 mit hoher Strahlungsintensität zu verwenden, beispielsweise ein Laser. Die Karieserreger reagieren empfindlich auf die Strahlung mit einer Wellenlänge im Bereich von 360 bis 420 nm und sterben ab. Der Ort der Kariesbehandlung kann durch das Filter 24 hindurch beobachtet werden.

In den folgenden Figuren werden weitere Ausführungsbeispiele dargestellt, bei denen gleiche Teile jeweils gleich bezeichnet sind. Als Strahlquelle 12 wird in Fig. 2 ein Laser verwendet. Seine monochromatische Strahlung 18 wird über den Lichtleiter 16 dem Zahn 20 zugeführt. Zusätzlich enthält die Beleuchtungseinrichtung 10 eine weitere Lichtquelle 29, die weißes Licht variabler Intensität erzeugt, das dem Strahl 18 über einen Teilerspiegel 31 zugemischt wird. Der Lichtleiter 16 ist von einem äußeren Lichtleiter 30 mit geordneten Faserbündeln umgeben, die der Bildübertragung dienen. Der Lichtleiter 16 ist gegenüber dem äußeren Lichtleiter 30 verschiebbar angeordnet, wie durch einen Pfeil 34 angedeutet ist. In einer zurückgezogenen Stellung, in der das dem Zahn 20 zugewandte Ende des Lichtleiters 16 nahe dem Ende des äußeren Lichtleiters 30 ist, beleuchtet die Strahlung 18 eine große Fläche des Zahns 20. In einer weiter zum Zahn 20 verschobenen Stellung des Lichtleiters 16 können einzelne Stellen des Zahns 20 näher untersucht werden. Beispielsweise ist es möglich, eine Kaverne 28 des Zahns 20 näher zu untersuchen und gegebenenfalls zu behandeln.

Die vom äußeren Lichtleiter 30 übertragene bildgebende Strahlung 22 wird durch das Filter 24 gefiltert und einer Kamera 32 zugeführt, mit der Aufnahmen vom Zahn 20 angefertigt werden können. Das von der weiteren Strahlungsquelle 29 emittierte Weißlicht dient zur Aufhellung des Hintergrunds, so daß die Konturen des Zahns 20 trotz Filterung durch das Filter 24 oder bei herausgeschwenktem Filter 24 noch sichtbar sind.

In Fig. 3 wird die von der Beleuchtungseinrichtung 10 ausgesendete Strahlung 18 durch eine Kollektorlinse 36 gebündelt und über einen teildurchlässigen Teilerspiegel 38 einem Bildleiter 40 zugeführt. Dieser besteht aus einem kohärenten oder geordneten Faserbündel, bei dem die einzelnen Fasern der Eintritts- und der Austrittsfläche des Bildleiters 40 in gleicher Weise angeordnet sind. Die Austrittsfläche des Bildleiters 40 ist mit einer Linse 42 versehen, die die Abbildungsfunktion des Bildleiters 40 verbessert. Die von einem Zahn (nicht dargestellt) zurückgestrahlte Strahlung 22 wird ebenfalls im Bildleiter 40 geführt und am Teilerspiegel 38 zu einer Feldlinse 44 ausgespiegelt, wobei das Filter 24 dem Teilerspiegel 38 nachgeordnet ist. Das vom Bildleiter 40 übertragene Bild kann von der Foto- oder Videokamera 32 aufgenommen werden. An die Stelle der Kamera 32 kann auch eine Detektorvorrichtung treten, die aus der ihr zugeführten Strahlung ein elektrisches Signal bildet. Anhand dieses Signals ist es möglich, das Ergebnis der Untersuchung an kariösen Zähnen zu quantifizieren.

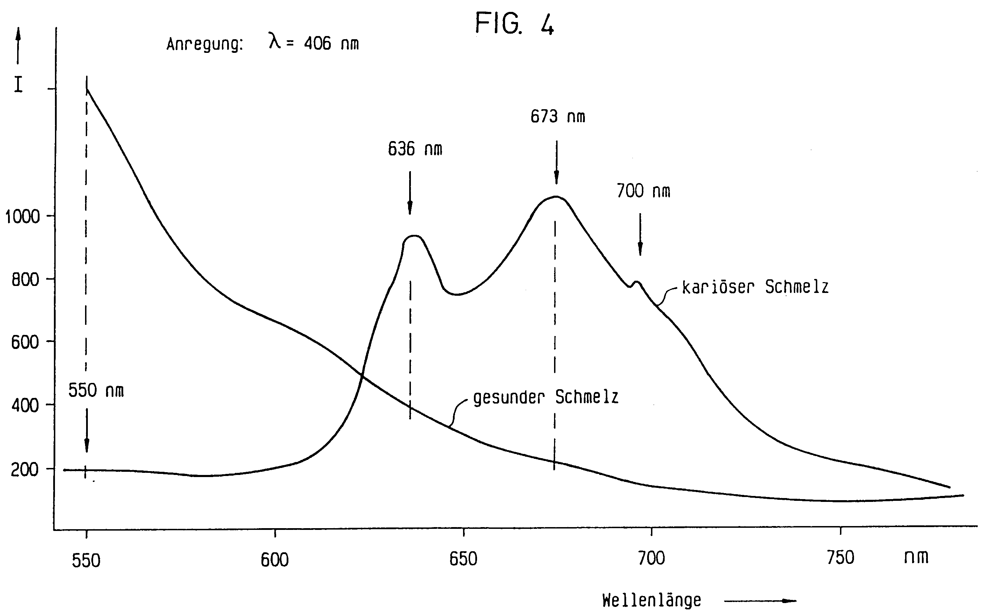

In Fig. 4 sind die Verläufe der von einem Zahn zurückgestrahlten Strahlungsintensität I in relativen Einheiten über der Wellenlänge in nm einmal für gesunden Schmelz und ein andermal für kariösen Schmelz aufgetragen. Die einfallende Strahlung, d.h. die Anregungsstrahlung, hat die Wellenlänge 406 nm. Wie dem Schaubild zu entnehmen ist, unterscheiden sich die dargestellten Verläufe. Insbesondere zeigt der Intensitätsverlauf für kariösen Schmelz drei Intensitätsmaxima bei 636, 673 und 700 nm. Das unterschiedliche Fluoreszenzverhalten von kariösem und gesundem Schmelz wird bei einem nachfolgend beschriebenen Ausführungsbeispiel der Erfindung zur qualitativen und quantitativen Erkennung von Karies ausgenutzt.

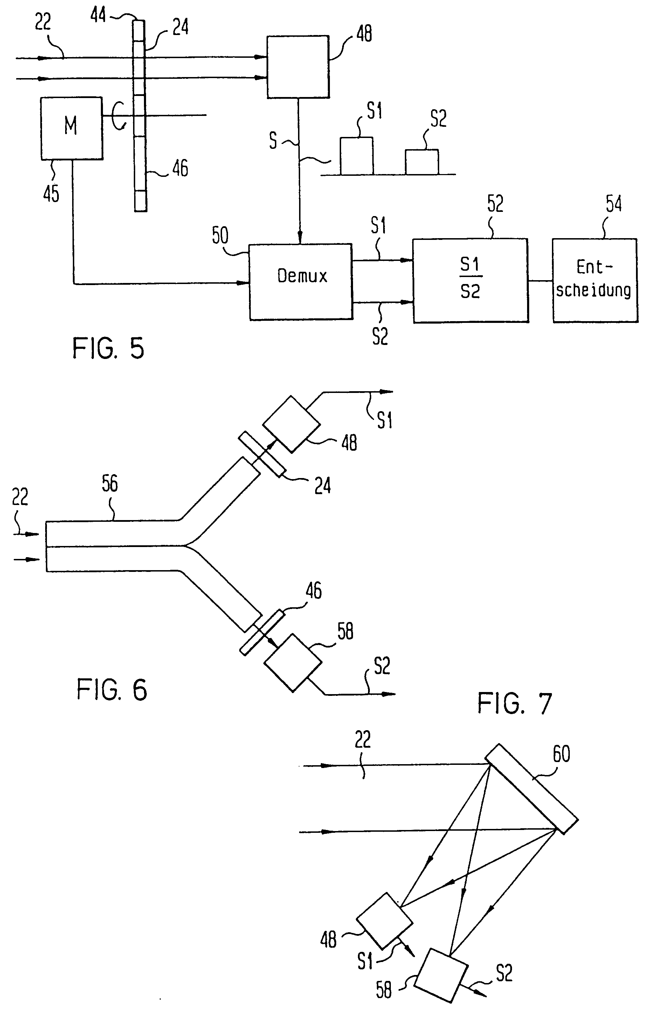

Bei der in Fig. 5 dargestellten Dektektionsvorrichtung sind auf einem Filterrad 44 das Filter 24 sowie ein weiteres Filter 46 angeordnet. Die Filter 24, 46 sind Schmalbandfilter und für Wellenlängen um 636 nm bzw. um 550 nm durchlässig. Bei Drehung des Filterrads 44 durch einen Motor 45 werden die Filter 24, 46 nacheinander in den Strahlengang der rückgestrahlten Strahlung 22 gebracht. Eine Fotodiode 48 wandelt die ihr zugeführte gefilterte Strahlung in ein elektrisches Signal S um, das zeitversetzte Teilsignale S1 und S2 enthält, die je einer von den Filtern 24 bzw. 46 durchgelassenen Strahlungsintensität proportional sind. Das Signal S wird einem Demultiplexer 50 zugeführt, der es synchron zur Drehung des Filterrads 44 abtastet und die Teilsignale S1, S2 aus dem Signal S abtrennt. Diese Teilsignale S1, S2 werden einem Quotientenbildner 52 zugeführt, der aus ihnen einen Quotienten bildet. Das Ergebnis wird einem Entscheidungsbaustein 54 zugeführt, wo es mit zuvor beispielsweise aus dem Schaubild nach Fig. 4 ermittelten Referenzkennwerten verglichen wird. Abhängig vom Vergleich wird dann angezeigt, ob die untersuchte Stelle des Zahns von Karies befallen ist oder nicht. Der Quotient selbst dient als Maß für den Kariesbefall.

In Fig. 6 ist eine andere Detektorvorrichtung dargestellt, bei der die zurückgestrahlte Strahlung 22 in einem Lichtleiter oder Lichtleiterbündel 56 geführt ist, der die Strahlung 22 aufteilt. Ein erster Teil dieser Strahlung 22 wird über das Filter 24 dem Detektor 48 zugeführt, der das Teilsignal S1 erzeugt. Der andere Teil der Strahlung 22 wird über das Filter 46 einem weiteren Detektor 58 zugeführt, der das Teilsignal S2 erzeugt. Die Teilsignale S1, S2 werden dann wie beim Ausführungsbeispiel nach Fig. 5 weiterverarbeitet.

In Fig. 7 ist in den Strahlengang der zurückgestrahlten Strahlung 22 ein Reflexionsgitter 60 eingeschaltet, das die Strahlung 22 abhängig von der Wellenlänge in verschiedene Richtungen reflektiert und bündelt. Die Detektoren 48, 58 sind so angeordnet, daß sie die Strahlung 22 bei den oben genannten Wellenlängen empfangen und die Teilsignale S1 und S2 erzeugen.

## Patentansprüche

1. Einrichtung zum Erkennen von Karies an Zähnen, mit einer Beleuchtungseinrichtung (10), die Strahlung einer vorgegebenen Wellenlänge auf mindestens einen Zahn (20) aussendet, und mit mindestens einem Filter (24, 46); das eine vom Zahn (20) zurückgestrahlte Strahlung (22) in einem vorgegebenen Wellenbereich durchläßt, wobei
- die durchgelassene Strahlung (22) zur Karieserkennung ausgewertet wird,
- und die Beleuchtungseinrichtung (10) mindestens einen Lichtleiter (16, 30, 40) hat, durch den die Strahlung (18) dem Zahn (20) zuführbar ist,
**dadurch gekennzeichnet,**
daß der Lichtleiter (40) auf der Seite der Beleuchtungseinrichtung (10) mit einem Teilerspiegel (38) versehen ist, dessen Teilerfläche gegen die optische Achse des Lichtleiters (40) geneigt ist, und der die zurückgestrahlte Strahlung (22) seitlich herausreflektiert.

2. Einrichtung nach Anspruch 1,
**dadurch gekennzeichnet,**
daß die Teilerfläche des Teilerspiegels (38) unter einem Winkel von 45° gegen die optische Achse des Lichtleiters (40) geneigt ist.

3. Einrichtung nach Anspruch 1 oder 2,
**dadurch gekennzeichnet,**
daß die Beleuchtungseinrichtung (10) Strahlung (18) im Wellenlängenbereich von 406 nm aussendet.

4. Einrichtung nach Anspruch 1 oder 2,
**dadurch gekennzeichnet,**
daß die Beleuchtungseinrichtung Strahlung (18) im Wellenlängenbereich von 360 bis 580 nm aussendet.

5. Einrichtung nach Anspruch 1 oder 2,
**dadurch gekennzeichnet,**
daß die Beleuchtungseinrichtung (10) Strahlung (18) im Wellenlängenbereich von 360 bis 420 nm oder im Wellenlängenbereich von 470 bis 580 nm aussendet.

6. Einrichtung nach einem der vorherigen Ansprüche,
**dadurch gekennzeichnet,**
daß der Filter (24) die zurückgestrahlte Strahlung (22) im Wellenlängenbereich größer als 620 nm durchläßt.

7. Einrichtung nach einem der vorhergehenden Ansprüche, dadurch **gekennzeichnet**, daß das Filter (24) die zurückgestrahlte Strahlung (22) im Wellenlängenbereich von 620 bis 720 nm durchläßt.

8. Einrichtung nach einem der vorhergehenden Ansprüche, dadurch **gekennzeichnet**, daß die Beleuchtungseinrichtung (10) als Strahlungsquelle (12) eine Quecksilberdampflampe, einen Kryptonlaser, eine Halogenlampe oder einen Farbstofflaser enthält, wobei in den Strahlengang der jeweiligen Strahlungsquelle (12) wahlweise ein Strahlungsfilter (14) zum Ausfiltern des jeweiligen Wellenlängenbereiches der Strahlung angeordnet ist.

9. Einrichtung nach einem der vorhergehenden Ansprüche, dadurch **gekennzeichnet**, daß das Filter (24) bzw. das Strahlungsfilter (14) als Absorptionsfilter, als Interferenzfilter, als Monochromator oder als Reflexionsfilter (60) ausgebildet ist.

10. Einrichtung nach Anspruch 9, dadurch **gekennzeichnet**, daß das Filter (24) auf einem Brillengestell angeordnet ist.

11. Einrichtung nach einem der vorhergehenden Ansprüche, dadurch **gekennzeichnet**, daß dem Filter (24) in Richtung der zurückgestrahlten Strahlung (22) gesehen ein Detektor (48) nachgeschaltet ist, der die ihm zugeführte Strahlung in ein erstes elektrisches Signal umwandelt.

12. Einrichtung nach Anspruch 11, dadurch **gekennzeichnet**, daß als Detektor (48) ein Sekundärelektronenvervielfacher oder eine Fotodiode vorgesehen ist.

13. Einrichtung nach einem der Ansprüche 11 oder 12, dadurch **gekennzeichnet**, daß ein weiteres Filter (46) vorgesehen ist, das die vom Zahn (20) zurückgestrahlte Strahlung (22) in einem weiteren, vom Wellenlängenbereich des Filters (24) verschiedenen Wellenlängenbereich durchläßt, und daß der Detektor (48) oder ein weiterer Detektor (58) die ausgefilterte Strahlung in ein zweites elektrisches Signal (S2) umwandelt, das einem Quotientenbilder (52) zugeführt wird, der aus dem ersten Signal (S1) und dem zweiten Signal (S2) einen Quotienten bildet, der als Maß für einen Kariesbefall dient.

14. Einrichtung nach Anspruch 13, dadurch **gekennzeichnet**, daß das Filter (24) und das weitere Filter (46) auf einer Zerhackerscheibe (44) angeordnet und bei Drehung der Zerhackerscheibe (44) nacheinander in den Strahlengang der zurückgestrahlten Strahlung (22) einschaltbar sind, daß der Detektor (48) die ausgefilterte Strahlung erfaßt, und daß aus dem vom Detektor (48) abgegebenen Signal (S) durch zur Drehung der Zerhackerscheibe (44) synchrones Abtasten das erste (S1) und zweite Signal (S2) erzeugt wird.

15. Einrichtung nach Anspruch 13, dadurch **gekennzeichnet**, daß die zurückgestrahlte Strahlung (22) von mindestens zwei Lichtleitfasern (56) erfaßt wird, die jeweils auf der dem Zahn abgewandten Ende mit dem Filter (24) bzw. dem weiteren Filter (46) versehen sind, wobei jedem Filter (24, 46) ein Detektor (48, 58) nachgeordnet ist, der die gefilterte Strahlung erfaßt.

16. Einrichtung nach einem der Ansprüche 13 bis 15, dadurch **gekennzeichnet**, daß das Filter (24) für Strahlung der Wellenlänge im Bereich um 636 oder 673 nm und das weitere Filter (46) für Strahlung der Wellenlänge im Bereich um 550 nm durchlässig sind.

17. Einrichtung nach einem der vorhergehenden Ansprüchen, dadurch **gekennzeichnet**, daß als Lichtleiter ein Bildleiter (40) vorgesehen ist.

18. Einrichtung nach einem der vorhergehenden Ansprüchen, dadurch **gekennzeichnet**, daß der Lichtleiter (16) von einem äußeren Lichtleiter (30) umgeben ist, der die zurückgestrahlte Strahlung (22) führt.

19. Einrichtung nach Anspruch 18, dadurch **gekennzeichnet**, daß der Lichtleiter (16) innerhalb des ihn umgebenden äußeren Lichtleiters (30) verschiebbar angeordnet ist.

20. Einrichtung nach einem der vorhergehenden Ansprüche, **gekennzeichnet** durch ein den oder die Lichtleiter aufnehmendes Endoskop, an das eine Kamera (32) anschließbar ist.

21. Einrichtung nach einem der vorhergehenden Ansprüche, dadurch **gekennzeichnet**, daß die Beleuchtungseinrichtung (10) eine weitere Lichtquelle (29) enthält, die weißes Licht erzeugt, das der ausgesendeten Strahlung (18) zumischbar ist.

## Claims

1. Device for detecting dental caries, having a lighting device (10) which emits radiation of a preset wavelength onto at least one tooth (20), and having at least one filter (24, 46) which transmits radiation reflected by the tooth (20) in a preset wave range, wherein
- the transmitted radiation (22) is evaluated to detect caries,
- and the lighting device (10) has at least one light guide (16, 30, 40), through which the radiation (18) may be supplied to the tooth (20),
**characterized in**
that the light guide (40) is provided, at the end nearer the lighting device (10), with a divider reflector (38) which has a divider surface inclined relative to the optical axis of the light guide (40) and which reflects the reflected radiation (22) laterally outwards.

2. Device according to claim 1,
**characterized in**
that the divider surface of the divider reflector (38) is inclined at an angle of 45° relative to the optical axis of the light guide (40).

3. Device according to claim 1 or 2,
**characterized in**
that the lighting device (10) emits radiation (18) in the wavelength range of 406 nm.

4. Device according to claim 1 or 2,
**characterized in**
that the lighting device emits radiation (18) in the wavelength range of 360 to 580 nm.

5. Device according to claim 1 or 2,
**characterized in**
that the lighting device (10) emits radiation (18) in the wavelength range of 360 to 420 nm or in the wavelength range of 470 to 580 nm.

6. Device according to one of the preceding claims,
**characterized in**
that the filter (24) transmits the reflected radiation (22) in the wavelength range greater than 620 nm.

7. Device according to one of the preceding claims, **characterized** in that the filter (24) transmits the reflected radiation (22) in the wavelength range of 620 to 720 nm.

8. Device according to one of the preceding claims, **characterized** in that the lighting device (10) has, as a radiation source (12), a mercury-vapour lamp, a krypton laser, a halogen lamp or a dye laser, a radiation filter being selectively disposed in the beam path of the respective radiation source (12) for filtering out the respective wavelength range of the radiation.

9. Device according to one of the preceding claims, **characterized** in that the filter (24) and/or the radiation filter (14) takes the form of an absorption filter, an interference filter, a monochromator or a reflection filter (60).

10. Device according to claim 9, **characterized** in that the filter (24) is disposed on a spectacle mount.

11. Device according to one of the preceding claims, **characterized** in that a detector (48) is disposed - viewed in the direction of the reflected radiation (22) - downstream of the filter (24) and converts the radiation supplied to it into a first electrical signal.

12. Device according to claim 11, **characterized** in that a secondary-emission multiplier or a photodiode is provided as detector (48).

13. Device according to one of claims 11 or 12, **characterized** in that a further filter (46) is provided, which transmits the radiation (22) reflected by the tooth (20) in a further wavelength range which differs from the wavelength range of the filter (24), and that the detector (48) or a further detector (58) converts the filtered-out radiation into a second electrical signal (S2) which is supplied to a quotient forming device (52), which from the first signal (S1) and the second signal (S2) forms a quotient which serves as a measure of a caries infection.

14. Device according to claim 13, **characterized** in that the filter (24) and the further filter (46) are disposed on a chopper disc (44) and during rotation of the chopper disc (44) may be successively introduced into the beam path of the reflected radiation (22), that the detector (48) detects the filtered-out radiation, and that from the signal (S) produced by the detector (48) the first (S1) and second signal (S2) is generated by scanning in synchronism with the rotation of the chopper disc (44).

15. Device according to claim 13, **characterized** in that the reflected radiation (22) is detected by at least two optical fibres (56) which, in each case at their end remote from the tooth, are provided with the filter (24) and the further filter (46) respectively, a detector (48, 58) which detects the filtered radiation being disposed downstream of each filter (24, 46).

16. Device according to one of claims 13 to 15, **characterized** in that the filter (24) may transmit radiation of the wavelength in the range around 636 or 673 nm and the further filter (46) may transmit radiation of the wavelength in the range around 550 nm.

17. Device according to one of the preceding claims, **characterized** in that an image guide (40) is provided as a light guide.

18. Device according to one of the preceding claims, **characterized** in that the light guide (16) is surrounded by a outer light guide (30) which carries the reflected radiation (22).

19. Device according to claim 18, **characterized** in that the light guide (16) is disposed displaceably inside the outer light guide (30) which surrounds it.

20. Device according to one of the preceding claims, **characterized** by an endoscope, which receives the light guide(s) and to which a camera (32) is connectable.

21. Device according to one of the preceding claims, **characterized** in that the lighting device (10) comprises a further light source (29) producing white light which may be added to the emitted radiation (18).

## Revendications

1. Dispositif pour détecter des caries dentaires, avec un dispositif d'éclairage (10) qui émet un rayonnement d'une longueur d'onde prédéterminée sur au moins une dent (20) et avec au moins un filtre (24, 46) qui transmet un rayonnement (22) rétro-réfléchi par une dent (20) dans un domaine de longueur d'onde prédéterminé,
- le rayonnement (22) transmis étant exploité aux fins de détecter des caries,
- et le dispositif d'éclairage (10) comprenant au moins une fibre optique (16, 30, 40) à l'aide de laquelle le rayonnement (18) peut être amené sur la dent (20),
caractérisé par le fait que la fibre optique (40), du côté du dispositif d'éclairage (10), est pourvu d'un miroir séparateur (38) dont la surface séparatrice est inclinée par rapport à l'axe optique de la fibre optique (40) et qui dévie vers l'extérieur le rayonnement (22) rétro-réfléchi.

2. Dispositif selon la revendication 1, caractérisé par le fait que que la surface séparatrice du miroir séparateur (38) est inclinée sous un angle de 45° par rapport à l'axe optique de la fibre optique (40).

3. Dispositif selon la revendication 1 ou 2, caractérisé par le fait que le dispositif d'éclairage (10) émet un rayonnement (18) dans le domaine de longueur d'onde de 406 nm.

4. Dispositif selon la revendication 1 ou 2, caractérisé par le fait que le dispositif d'éclairage émet un rayonnement (18) dans le domaine de longueur d'onde allant de 360 à 580 nm.

5. Dispositif selon la revendication 1 ou 2, caractérisé par le fait que le dispositif d'éclairage (10) émet un rayonnement (18) dans le domaine de longueur d'onde allant de 360 à 420 nm ou dans le domaine de longueur d'onde allant de 470 à 580 nm.

6. Dispositif selon une des revendications précédentes, caractérisé par le fait que le filtre (24) transmet le rayonnement rétro-réfléchi (22) dans le domaine de longueur d'onde supérieur à 620 nm.

7. Dispositif selon une des revendications précédentes, caractérisé par le fait que le filtre (24) transmet le rayonnement rétro-réfléchi (22) dans le domaine de longueur d'onde allant de 620 à 720 nm.

8. Dispositif selon une des revendications précédentes, caractérisé par le fait que le dispositif d'éclairage (10) comporte comme source de rayonnement (12) une lampe à vapeur de mercure, un laser à krypton, une lampe halogène ou un laser à colorants organiques, un filtre de rayonnement (14) destiné à sélectionner le domaine de longueur d'onde correspondant du rayonnement étant disposé de manière sélective sur le trajet du rayonnement de la source de rayonnement (12) concernée.

9. Dispositif selon une des revendications précédentes, caractérisé par le fait que le filtre (24) ou le filtre de rayonnement (14) est agencé sous forme de filtre absorbant, de filtre d'interférence, de monochromateur ou de filtre à réflexion (60).

10. Dispositif selon la revendication 9, caractérisé par le fait que le filtre (24) est disposé sur une monture de lunettes.

11. Dispositif selon une des revendications précédentes, caractérisé par le fait que, vu dans la direction du rayonnement rétro-réfléchi (22), un détecteur (48) est placé derrière le filtre (24), lequel détecteur transforme en un premier signal électrique le rayonnement qui lui est transmis.

12. Dispositif selon la revendication 11, caractérisé par le fait qu'il est prévu comme détecteur (48) un multiplicateur d'électrons secondaires ou une photodiode.

13. Dispositif selon une des revendications 11 ou 12, caractérisé par le fait qu'il est prévu un filtre supplémentaire (46) qui transmet le rayonnement (22) rétro-réfléchi par la dent (20) dans un domaine de longueur d'onde supplémentaire, différent du domaine de longueur d'onde du filtre (24), et par le fait que le détecteur (48) ou un détecteur supplémentaire (58) transforme le rayonnement sélectionné en un deuxième signal électrique (S2) qui est envoyé à un circuit diviseur (52) qui forme à partir du premier signal (S1) et du deuxième signal (S2) un quotient servant d'indication pour une carie.

14. Dispositif selon la revendication 13, caractérisé par le fait que le filtre (24) et le filtre supplémentaire (46) sont disposés sur un disque hacheur (44) et, lors de la rotation du disque hacheur (44), peuvent être amenés successivement sur le trajet du rayonnement rétro-réfléchi (22), par le fait que le détecteur (48) détecte le rayonnement transmis et par le fait qu'à partir du signal (S) délivré par le détecteur (48), par analyse synchronisée avec le disque hacheur (44), on produit les premier (S1) et deuxième (S2) signaux.

15. Dispositif selon la revendication 13, caractérisé par le fait que le rayonnement rétro-réfléchi (22) est détecté par au moins deux fibres otpiques (56) qui, à leur extrémité éloignée de la dent, sont munies respectivement du filtre (24) et du filtre supplémentaire (46), un détecteur (48, 58) qui détecte le rayonnement transmis étant associé à chacun des filtres (24, 46).

16. Dispositif selon une des revendications 13 à 15, caractérisé par le fait que le filtre 24 transmet le rayonnement dont la longueur d'onde se situe dans la plage autour de 636 ou de 673 nm et le filtre supplémentaire (46) le rayonnement dont la longueur d'onde se situe dans la plage autour de 550 nm.

17. Dispositif selon une des revendications précédentes, caractérisé par le fait qu'il est prévu comme fibre optique une fibre vidéo (40).

18. Dispositif selon une des revendications précédentes, caractérisé par le fait que la fibre optique (16) est entourée d'une fibre optique extérieure (30) qui achemine le rayonnement rétro-réfléchi (22).

19. Dispositif selon la revendication 18, caractérisé par le fait que la fibre optique (16) est disposée à l'intérieur de la fibre optique extérieure (30) qui l'entoure.

20. Dispositif selon une des revendications précédentes, caractérisé par un endoscope recevant la ou les fibres optiques, auquel une caméra (32) peut être connectée.

21. Dispositif selon une des revendications précédentes, caractérisé par le fait que le dispositif d'éclairage (10) comporte une source lumineuse (29) supplémentaire qui produit de la lumière blanche pouvant être mélangée au rayonnement (18) émis.
